(19) Europäisches Patentamt
European Patent Office
Office européen des brevets

(11) Veröffentlichungsnummer: **0 002 708**
**B1**

(12) **EUROPÄISCHE PATENTSCHRIFT**

(21) Anmeldenummer: **78101626.6**

(22) Anmeldetag: **09.12.78**

(51) Int. Cl.³: **C 07 C 118/04,**
**C 07 C 119/00**

# (54) Verfahren zur kontinuierlichen Herstellung von Monoisocyanaten

(30) Priorität: **21.12.77 DE 2756928**

(43) Veröffentlichungstag der Anmeldung:
**11.07.79 Patentblatt 79/14**

(45) Bekanntmachung des Hinweises auf die Patenterteilung:
**15.10.80 Patentblatt 80/21**

(84) Benannte Vertragsstaaten:
**BE CH DE FR GB IT NL**

(56) Entgegenhaltungen:
**DE - A - 1 944 719**
**DE - A - 2 512 514**
**DE - A - 2 635 490**
**US - A - 3 076 007**
**US - A - 3 919 279**
**US - A - 3 962 302**
**US - A - 3 992 430**

(73) Patentinhaber: **BAYER AG**
**Zentralbereich Patente, Marken und Lizenzen**
**D - 5090 Leverkusen 1**
**Bayerwerk (DE)**

(72) Erfinder: **Reichmann, Wolfgang, Dr.**
**Vohwinkelallee 19**
**D - 4000 Düsseldorf (DE)**
**König, Klaus, Dr.**
**Heymannstrasse 50**
**D - 5090 Leverkusen (DE)**
**Koster, Johannes, Dr.**
**Schulstrasse 46**
**D - 4047 Dormagen 5 (DE)**

**EP 0 002 708 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Verfahren zur kontinuierlichen Herstellung von Monoisocyanaten

Die Erfindung betrifft ein verbessertes Verfahren zur kontinuierlichen Herstellung von aliphatischen Monoisocyanaten aus den entsprechenden N-Alkylcarbamidsäurearylestern.

Isocyanate werden bekanntlich durch Umsetzung von Aminen mit Phosgen hergestellt. Die Reaktion verläuft über die Stufe der Carbamidsäurechloride, die bei höherer Temperatur in die entsprechenden Isocyanate und Chlorwasserstoff spalten. Liegt der Siedepunkt des herzustellenden Isocyanats deutlich über der Zersetzungstemperatur des Carbamidsäurechlorids, so kann der bei der Zersetzung gebildete Chlorwasserstoff, insbesondere bei Verwendung eines inerten organischen Lösungsmittels, problemlos aus dem Reaktionsraum entfernt werden. Wenn jedoch die Zersetzungstemperatur des Carbamidsäurechlorids in der Nähe oder oberhalb des Siedepunkts des Isocyanats liegt, so gelangt das Isocyanat in den Gasraum, wo es mit dem Chlorwasserstoff zu Carbamidsäurechlorid rekombiniert. Die Spaltung verläuft daher unvollkommen; das Isocyanat wird nur in geringer Ausbeute und mit Carbamidsäurechlorid verunreinigt erhalten.

Der geschilderte Sachverhalt trifft insbesondere für aliphatische Monoisocyanate zu, deren aliphatische Reste 1 bis 3 Kohlenstoffatome enthalten, wobei die Schwierigkeiten bei der Herstellung von Methylisocyanat am größten sind.

In der Patentliteratur sind zahlreiche Verfahren beschrieben, mit denen die genannten Schwierigkeiten überwunden werden sollen. Ein Großteil dieser Verfahren beschreibt die Spaltung von Carbamidsäurechloriden unter Verwendung von Chlorwasserstoffakzeptoren.

So ist es z.B. bekannt, Isocyanate aus Carbamidsäurechloriden in Gegenwart von organischen Basen (z.B. tertiären Aminen) oder Carbonsäure-dialkylamiden (DT—OS 1 593 554) oder Tetraalkylharnstoffen (US—PS 3 644 461) in organischen Lösungsmitteln herzustellen. Ferner wird zur Absorption des Chlorwasserstoffs die Verwendung von Wasser (DT—AS 2 156 761) und von wäßrigen Lösungen oder Suspensionen anorganischer Basen (GB—PS 1 208 862) beschrieben. Auch Olefine werden als Chlorwasserstoffakzeptoren genannt (DT—OS 2 210 285).

Alle diese Verfahren weisen den gravierenden Nachteil auf, daß Nebenprodukte entstehen (korrosive organische oder anorganische Salze oder Alkylchloride), die entweder kostspielig aufgearbeitet werden müssen oder aber eine Umweltbeeinträchtigung darstellen. Darüber hinaus besteht bei Verwendung von organischen Basen die Gefahr von Nebenreaktionen zu di- und trimeren Isocyanaten. In Gegenwart von Wasser wird ein beträchtlicher Teil des Carbamidsäurechlorids zum Aminhydrochlorid hydrolysiert, so daß nur im Falle des reaktionsträgen tertiären Butylisocyanats gute Ausbeuten erzielt werden.

Auch die Herstellung von niedrigsiedenden aliphatischen Monoisocyanaten durch thermische Spaltung von Carbamidsäurechloriden in organischen Lösungsmitteln unter Anwendung spezieller Verfahrenstechniken ist bekannt.

Nach DT—AS 1 193 034 wird die thermische Spaltung des Carbamidsäurechlorids in einem mit Rückflußkühler und trennwirksamer Kolonne versehenen Reaktor durchgeführt. Durch den Rückflußhler entweicht Chlorwasserstoff, während Isocyanat, Carbamidsäurechlorid und Lösungsmittel zurückgehalten werden. Das gebildete Isocyanat gelangt in die Kolonne und kann am Kolonnenkopf abgenommen werden. Durch einen Rücklaufteiler wird der größte Teil des Isocyanats zurückgeführt, damit in die Kolonne aufsteigender Chlorwasserstoff vollständig absorbiert wird und in Form von Carbamidsäurechlorid in den Reaktor zurückgelangt.

Bei der kontinuierlichen Durchführung dieses Verfahrens wird an Carbamidsäurechlorid verarmte Lösung laufend dem Reaktor entnommen, an anderer Stelle mit Carbamidsäurechlorid angereichert und dem Reaktor wieder zugeführt.

Auch die deutschen Offenlegungsschriften 2 411 441, 2 411 442, 2 422 211 und 2 503 270 betreffen die thermische Spaltung von Carbamidsäurechloriden unter Verwendung spezieller Vorichtungen.

Zwar wird durch die genannten Verfahren die Herstellung von niedrigsiedenden aliphatischen Monoisocyanaten durch thermische Spaltung von Carbamidsäurechloriden ermöglicht, jedoch sind auch hier schwerwiegende Nachteile zu nennen:

1. Die Chlorwasserstoffabtrennung erfordert Rückflußkühler mit großen Kühlflächen, die in energetisch aufwendiger Weise mit Kältemittel betrieben werden müssen, damit Isocyanat und Carbamidsäurechlorid quantitativ zurückgehalten werden.
2. Zur destillativen Abtrennung von Carbamidsäurechloridfreiem Isocyanat aus dem Reaktionsgemisch werden hochwirksame Fraktionierkolonnen benötigt, wobei ein hohes Rücklaufverhältnis eingestellt werden muß.
3. Die Verfahren lassen sich nur günstig durchführen, wenn verhältnismäßig verdünnte Carbamidsäurechloridlösungen (1 bis 30%ig) eingesetzt werden.
4. Bei der kontinuierlichen Durchführung (großtechnisch kommt nur eine solche in Frage) muß die Reaktionslösung mehrfach im Kreis geführt werden.

All dies hat zur Folge, daß die Reaktionskomponenten (Isocyanat, Carbamidsäurechlorid und Lösungsmittel) im Prozeß mehrmals verdampft und kondensiert, bzw. abgekühlt und wieder aufgeheizt werden müssen, wodurch ein hoher Energieverbrauch verursacht wird. Aus dem Einsatz von verdünnten Lösungen und der Notwendigkeit der vielen Kreisläufe resultiert eine lange Verweilzeit und somit einne niedrige Raum-Zeit-Ausbeute. Durch die lange Verweilzeit besteht die Gefahr der Ausbeuteminderung durch Trimerisierung des Monoisocyanats. Der Prozeß erfordert einen hohen Aufwand an Meß- und Regeltechnik. Hieraus, aus der niedrigen Raum-Zeit-Ausbeute und der Nitwendigkeit des Einsatzes von hochwirksamen Fraktionierkclonnen ergibt sich für die technische Produktion ein hoher Investitionsaufwand.

Ferner ist bekannt, Isocyanate durch thermische Spaltung von Carbamidsäureestern herzustellen (Houben-Weyl, Methoden der org. Chemie.; Bd. 8, Seite 126, 1952). Hierbei setzt man bevorzugt Carbamidsäurearylester ein, da diese im Vergleich zu Alkylestern unter milderen Bedingungen Isocyanate abspalten.

In den Patentliteratur sind Herstellungsverfahren für Moniisocyanate beschrieben, bei denen sowohl Carbamidsäurearyl- als auch alkylester verwendet werden.

So lassen sich gemäß der US—PS 3 076 007 aus N-Alkylcarbamidsäure-2-hydroxyäthylestern und gemäß der DT—OS 2 512 514 aus N-Alkylcarbamidsäure-$\beta$-naphthylestern durch thermische Spaltung die entsprechenden Monoisocyanate freisetzen.

Hierbei besteht, wie bei der thermischen Spaltung von Carbamidsäurechloriden, die Gefahr, daß die Spaltprodukte wieder zu den Ausgangsstoffen rekombinieren.

Nach dem in der US—PS 3 076 007 beschriebenen Verfahren wird die Rekombination dadurch verhindert, daß die Spaltprodukte zusammen kondensiert und sofort abgeschreckt werden. Die erhaltenen Destillate werden dann mit einem mit Wasser nicht mischbaren inerten Lösungsmittel verdünnt. Das Äthylenglykol wird durch mehrmalige Extraktion mit Wasser entfernt.

Diese umständliche Trennung der Spaltprodukte durch Extraktion ist bei dem in der DT—OS 2 512 514 beschriebenen Verfahren nicht notwendig, da das bei der Spaltung entstehende $\beta$-Naphthol unter den angegebenen Zersetzungsbedingungen aufgrund seines hohen Siedepunktes nicht in die Gasphase übergeht.

Hierbei ist jedoch die permanente thermische Belastung des $\beta$-Naphthols von Nachtiel, durch die die Bildung von unerwünschten Pyrolyseprodukten hervorgerufen wird. Dieser Tatbestand wirkt sich insbesondere bei der kontinuierlichen Durchführung des Verfahrens negativ aus, da das wieder eingesetzte $\beta$-Naphthol mit Verunreinigungen angereichert wird, die zwangsläufig den Herstellungsprozeß der Isocyanate stören.

Ein weiterer Nachteil des Verfahrens liegt darin begründet, daß mit fortschreitender Zersetzung der Carbamidsäureester eine Verlangsamung der Isocyanatabtrennung in der Weise auftritt, wie die mit der Zersetzung einhergehende Konzentrationszunahme an $\beta$-Naphthol das Gleichgewicht zwischen $\beta$-Naphthol, Isocyanat und Carbamidsäureester nach dem Massenwirkungsgesetz verschiebt.

Auch die US—PS 3 962 302 befaßt sich mit der Herstellung von Isocyanaten durch thermische Spaltung der entsprechenden Carbamidsäureester, wobei auch O-arylsubstituierte Carbamidsäurester zum Einsatz gelangen können. Bei diesem Verfahren erfolgt die Spaltung in Gegenwart eines unter den Reaktionsbedingungen nicht flüchtigen Lösungsmittels, wobei lediglich eines der Spaltprodukte Hydroxylverbindung bzw. Isocyanat) dem flüssigen Reaktionsmedium während der Spaltreaktion entweicht. Dieses Verfahren ist offensichtlich zur Herstellung von Isocyanaten in hohen Ausbeuten nicht geeignet, da in den Ausführungsbeispielen deutlich unter 100% liegende Ausbeuten mitgeteilt werden, wobei sich diese Ausbeuteangaben lediglich auf umgesetztes und nicht auf eingesetztes Ausgangsmaterial beziehen.

Aufgabe der vorliegenden Erfindung war es nun, ein Verfahren zur kontinuierlichen Herstellung von Monoisocyanaten zu finden, bei dem unerwünschte Rekombinationen, Trennprobleme und unnötige thermische Belastungen der durch thermische Zersetzung von N-Alkylcarbamidsäurearylestern entstandenen Spaltprodukte vermieden werden.

Diese Aufgabe konnte durch das nachstehend beschriebene erfindungsgemäße Verfahren gelöst werden.

Gegenstand der vorliegenden Erfindung ist ein Verfahren zur kontinuierlichen Herstellung von Monoisocyanaten der Formel

R—NCO

in welcher

R für einen gesättigten aliphatischen Kohlenwasserstoffrest mit 1 bis 3 Kohlenstoffatomen steht, durch thermische Spaltung der entsprechenden, in einem unter den Reaktionsbedingungen interten organischen Lösungsmittel mit einem mindestens 20°C über dem Siedepunkt des zu bildenden Monoisocyanats liegenden Siedepunkt gelösten N-Alkylcarbamidsäurearylester der Formel

R—NH—CO—O—R′

in welcher
R die genannte Bedeutung hat und
R' für einen Rest steht wie er durch Entfernung der Hydroxylgruppe aus einem Monophenol eines bei Normaldruck unterhalb 250°C liegenden Siedepunkts erhalten wird, und destillative Auftrennung der Spaltprodukte, dadurch gekennzeichnet, daß

a) man die Lösung des zu spaltenden N-Alkylcarbamidsäurearylesters bei Normaldruck oder bei leicht vermindertem Druck im Bereich von 200 bis 1013 mbar unter Spaltung des Esters in das entsprechende Monoisocyanat und das entsprechende Phenol, sowie unter Verdampfen des Lösungsmittels und der Spaltprodukte auf eine Temperatur im Bereich von 160 bis 250°C erhitzt und
b) man die gemäß a) anfallenden Dämpfe gemeinsam in eine Destillationskolonne leitet, aus welcher man das Phenol und zumindest die Hauptmenge des Lösungsmittels im Form eines gemeinsamen oder in Form mehrerer Seitenströme und das Monoisocyanat gegebenenfalls zusammen mit einem Teil des Lösungsmittels am Kopf gewinnt.

Gegenstand der vorliegenden Erfindung ist insbesondere auch eine Ausführungsform des erfindungsgemäßen Verfahrens, welche dadurch gekennzeichnet ist, daß man das als Seitenstrom anfallende Phenol und das als Seitenstrom anfallende Lösungsmittel im Gemisch oder nach destillativer Auftrennung zur Herstellung der als Ausgangsmaterial eingesetzten Lösung durch Auflösen von bzw. Reaktion mit bei der Gasphasenphosgenierung von Monoaminen der Formel $R—NH_2$ entstehenden Reaktionsgasen wiederverwendet, wobei R die obengenannte Bedeutung hat.

Ausgangsverbindungen für das erfindungsgemäße Verfahren sind N-Alkylcarbamidsäurearylester der Formel

$$R—NH—CO—O—R'$$

in welcher
R die bereits oben angegebene Bedeutung hat und vorzugsweise für eine Methylgruppe steht und
R' die bereits oben angegebene Bedeutung hat und vorzugsweise für einen gegebenenfalls Methyl-, Äthyl-, Methoxyoder Äthoxy-substituierten Phenylrest, besonders bevorzugt für einen unsubstituierten oder einfach Methylsubstituierten Phenylrest steht.

Derartige N-Alkylcarbamidsäurearylester werden nach bekannten Methoden des Standes der Technik hergestellt. Sie können z.B. durch Umsetzung von Alkylaminen mit Chlorameisensäurearylestern oder Diarylcarbonaten erhalten werden.

In sehr einfacher Weise werden diese, beim erfindungsgemäßen Verfahren als Ausgangsmaterialien einzusetzenden, Ester jedoch durch Reaktion von N-Alkylcarbamidsäurechloriden, die durch Phosgenierung der entsprechenden Amine $R—NH_2$ in der Gasphase entstehen, mit Phenolen der Formel

$$R'—OH$$

unter HCl-Abspaltung hergestellt, wobei

R' die bereits genannte Bedeutung hat.

Die beim erfindungsgemäßen Verfahren als Ausgangsmaterialien einzusetzenden N-Alkylcarbamidsäurearylester werden beim erfindungsgemäßen Verfahren in Form ihrer Lösungen in geeigneten inerten Lösungsmitteln eingesetzt.

Dementsprechend erfolgt vorzugsweise die Umsetzung der durch Gasphasenphosgenierung der Amine entstehend Reaktionsgase mit dem Phenol R'—OH in Gegenwart eines inerten organischen Lösungsmittels und zwar

a) entweder direkt durch Absorption der aus der Gasphase austretenden Reaktionsgase unter Abtrennung von Chlorwasserstoff und überschüssigem Phosgen mit der Phenollösung selbst oder
b) erst nach der Absorption der Reaktionsgase mit einem inerten, organischen Lösungsmittel und gegebenenfalls Entfernung von überschüssigem Phosgen, wobei sowohl das Carbamidsäurechlorid als auch das schon zu einem Teil frei vorliegende Isocyanat in den entsprechenden Carbamidsäurearylester übergeführt werden oder
c) nach Absorption der Reaktionsgase gemäß b) erst nach der Destillation der isocyanathaltigen Carbamidsäurechloric-Lösung in einer Kolonne unter Entnahme des Carbamidsäurechlorids zu sammen mit Lösungsmittel in einem Seitenstrom der Kolonne, wobei am Kopf der Kolonne das frei vorliegende Isocyanat und im Sumpf der Kolonne der Hauptteil des Lösungsmittels abgetrennt werden.

Die Variante c) hat erstens den Vorteil, daß das in den Absorptionslösungen der Gasphasenphosgenierung schon enthaltene Monoisocyanat direkt isoliert und nicht unnötigerweise zusammen mit dem Carbamidsäurechlorid zu Carbamidsäurearylester umgesetzt wird. Zweitens liefert die Seitenstromentnahme der beschriebenen Destillation eine Carbamidsäurechloridlösung, deren Konzentration in für das erfindungsgemäße Verfahren vorteilhafter Weise erhöht ist, im Vergleich zu den relativ niedrigen Carbamidsäurechlorid-Konzentrationen, die die Absorptionslösungen der Gasphasenphosgenierung im allgemeinen aufweisen.

Die Konzentrationen der für das erfindungsgemäße Verfahren eingesetzten N-Alkylcarbamidsäurearylester-Lösungen betragen bei Verwendung der vorgenannten Carbamidsäurechlorid-Lösungen 5—70 Gew.-%, vorzugsweise 40 bis 60 Gew.-%.

Erfindungsgemäß geeignete Phenole R′—OH sind z.B. Phenol selbst. o-, m- oder p-Kresol, die entsprechenden isomeren Äthylphenole, die verschiedenen isomeren Xylenole oder Alkoxyphenole wie o-Methoxyphenol oder p-Äthoxyphenol.

Natürlich können auch Gemische der beispielhaft genannten Phenole verwendet werden. Das unsubstituierte Phenol sowie die genannten Kresole sind bevorzugt. Phenol ist besonders bevorzugt.

Bei den beim erfindungsgemäßen Verfahren einzusetzenden Lösungsmitteln handelt es sich um solche, die

a) unter den erfindungsgemäßen Reaktionsbedingungen inert sind und
b) mindestens 20°C, bevorzugt mindestens 50°C oberhalb der erfindungsgemäß hergestellten Isocyanate sieden,

wobei der Siedepunkt der Lösungsmittel im allgemeinen nicht höher als der Siedepunkt der Phenole, vorzugsweise jedoch mindestens 20°C unterhalb und besonders bevorzugt mindestens 50°C unterhalb dem Siedepunkt der eingesetzten Phenole liegt.

Beispiele geeigneter Lösungsmittel sind:
n-Octan, Cyclohexylchlorid, Dichlorpropan-1,3, isomere Dichlorbutane, Toluol, Xylole, Äthylbenzol, Chlorbenzol, Dichlorbenzol, Essigsäurebutylester, Propionsäurepropylester.

Selbstverständlich können auch Gemische aus den genannten Lösungsmitteln verwendet werden. Chlorbenzol ist das bevorzugte Lösungsmittel.

Das erfindungsgemäße Verfahren sei im folgenden anhand der Zeichnungen näher erläutert.

Fig. 1 zeigt eine Destillationskolonne zur Durchführung des erfindungsgemäßen Verfahrens. Hierbei bedeuten

(101) ein Reaktionsgefäß zur thermischen Spaltung der Esterlösung;
(102) den unteren Teil einer auf das Reaktionsgefäß aufgesetzten Füllkörperkolonne;
(103) einen "Seitenstrom-Entnahmeboden";
(104) einen temperaturregulierbaren Dephlegmator;
(105) den oberen Teil der Füllkörperkolonne;
(106) den Zulauf für die zu spaltende Esterlösung;
(107) die Seitenstromentnahme und
(108) die Kopfprodukt-Entnahme.

Bei der Durchführung des erfindungsgemäßen Verfahrens wird nun über (106) die zu spaltende Lösung des N-Alkylcarbamidsäurearylesters dem Reaktionsgefäß (101) zugeführt, in dem bei einer Temperatur von 160—250°C, vorzugsweise 190—250°C und insbesondere 200—230°C thermische Spaltung des Carbamidsäureesters zu Isocyanat und Phenol eintritt, wobei die Spaltprodukte sofort nach ihrer Entstehung zusammen mit dem Lösungsmittel verdampfen und über den unteren Teil einer Füllkörperkolonne (102) den Dephlegmator (104) erreichen, dessen Temperatur so geführt wird, daß die Gesamtmenge des Phenols und der größte Teil des Lösungsmittels kondensiert und am Seitenstromentnahme-Boden (103) in Form des Stroms (107) der Kolonne entnommen werden kann. Die Gesamtmenge des Verfahrensprodukts wird der Kolonne gegebenenfalls zusammen mit geringen Anteilen an Lösungsmittel über Kopf (108) entnommen und gegebenenfalls einer Reindestillation zugeführt.

Die in Fig. 1 dargestellte Apparatur stellt eine bevorzugt zur Durchführung des erfindungsgemäßen Verfahrens geeignete Vorrichtung dar. Das erfindungsgemäße Verfahren ist jedoch keineswegs auf die zwingende Verwendung der in Fig. 1 dargestellten Vorrichtung beschränkt. So wäre es prinzipiell auch möglich, das erfindungsgemäße Verfahren unter Entnahme mehrerer Seitenströme, d.h. unter Verwendung einer zur Entnahme mehrerer Seitenströme geeigneten Kolonne durchzuführen, wobei die Hauptmenge des Phenols in einem unteren Seitenstrom und die Hauptmenge des Lösungsmittels in einem oberen Steitenstrom entnommen werden könnte. Die Durchführung des erfindungsgemäßen Verfahren unter Entnahme mehrerer Seitenströme ist insbesondere für die erfindungsgemäße Herstellung höherer Monoisocyanate (Äthylisocyanat und Propylisocyanate) geeignet. In Folge der höher liegenden Siedepunkte dieser Isocyanate wird das Temperaturprofil in der Destillationskolonne angehoben. Dadurch entsteht die Gefahr, daß mit dem Isocyanat geringe Anteile des Phenols den Kopf

der Destillationskolonne erreichen. Dieser Gefahr kann durch die mit Hilfe des Dephlegmators mögliche Temperaturegulierung entgegengewirkt werden. Die Dephlegmatortemperatur beeinflußt jedoch auch die Isocyanatmenge, die in die entnommene Phenollösung (107) gelangt. Da diese Menge minimal sein soll, kann die Dephlegmatortemperatur nicht beliebig gesenkt werden. Es ist daher günstiger, die Temperatur des Dephlegmators so einzustellen, daß die in die Phenollösung gelangende Isocyanatmenge möglichst klein ist. Das den Dephlegmator passierende Phenol wird dann mit geringen Anteilen an Isocyanat und Carbamidsäurearylester als Lösung in einer Teilmenge des verwendeten Lösungsmittels in einem zweiten Seitenstrom (nicht gezeichnet) an einer geeigneten Stelle oberhalb des Dephlegmators der Destillationskolonne entnommen und an einer beliebigen Stelle unterhalb der Entnahmestelle (107) in die Apparatur zurückgeführt. Durch die bei der Herstellung der höheren Monoisocyanate in der Destillationskolonne herrschenden höheren Temperaturen besteht weiterhin die Gefahr, daß die Spaltprodukte rekombinieren. Zur Verhinderung einer solchen Rekombination kann deshalb die Spaltung der Carbamidsäurearylester auch bei leicht vermindertem Druck innerhalb des Druckbereichs von 200 bis 1013 mbar durchgeführt werden. Zur teilweisen Kondensation der in der Kolonne aufsteigenden Dämpfe ist außerdem die Verwendung des in Fig. 1 dargestellten Dephlegmators nicht zwingend erforderlich; vielmehr kann die Kondensation auch durch eine andersartige Wärmeabführung wie z.B. Wärmeabstrahlung gewährleistet werden. Wesentlich zur Durchführung des erfindungsgemäßen Verfahrens ist, daß die Gesamtmenge des Lösungsmittels und der Spaltprodukte verdampft und daß das Blockierungsmittel (Phenol) in Form eines Seitenstroms und das Endprodukt (Monoisocyanat) am Kopf der Kolonne entnommen werden. Im übrigen erfolgt die Temperaturführung in der Kolonne im allgemeinen dergestalt, daß die Hauptmenge, d.h. mehr als 50 vorzugsweise mindestens 75% der Gesamtmenge des eingebrachten Lösungsmittels in Form eines (oder mehrerer) Seitenströme entnommen wird, so daß im Kopfprodukt allenfalls bis zu 50 bzw. 25% der Gesamtmenge des eingetragenen Lösungsmittels vorliegen.

Mit den Fig. 2 bis 4 werden denkbare Ausführungsformen des erfindungsgemäßen Verfahrens erläutert, wobei die in Fig. 1 im Detail dargestellte Kolonne jeweils mit "A" bezeichnet wurde.

Fig. 2 zeigt neben dieser Kolonne A eine Auswaschkolonne B. Bei der Durchführung des erfindungsgemäßen Verfahrens gemäß der in Fig. 2 gezeigten Ausführungsform gelangen die einen Gasphasenphosgenierreaktor (nicht gezeichnet) verlassenden Reaktionsgase (201) in die Auswaschkolonne B, in welcher sie mit einer Lösung eines Phenols in einem inerten Lösungsmittel (207) vereinigt und zur Reaktion gebracht werden. Die Vereinigung des gegebenenfalls im oberen Teil der Kolonne B gekühlten Gasstroms mit der Lösung (207) erfolgt im allgemeinen im Temperaturbereich zwischen 20 und 150°C. Durch Erhitzen der entstehenden Lösung (Sumpf der Kolonne B) wird überschüssiges Phosgen und durch die Reaktion zwischen Carbamidsäurechlorid und Phenol entstehender Chlorwasserstoff ausgetrieben und über (202) entfernt. Die dem Sumpf der Kolonne B entnommene Lösung (206) stellt das eigentliche Ausgangsmaterial zur Durchführung des erfindungsgemäßen Verfahrens dar. Der Seitenstrom (207) entspricht dem Seitenstrom (107) und stellt eine die Gesamtmenge des Phenols enthaltende Lösung dar, die ohne weitere Aufarbeitung in die Auswaschkolonne B zurückgeführt wird. Das Verfahrensprodukt wird in Form des Stroms (208) am Kopf der Kolonne A gewonnen, wobei es sich bei dem Strom (208) auch um eine Lösung des Isocyanats in Restmengen an Lösungsmittel handeln kann, die in einer weiteren Destillationskolonne (nicht gezeichnet) in reines Verfahrensprodukt und wiederverwendbares Lösungsmittel aufgetrennt werden kann.

Die Vorrichtung gemäß Fig. 3 unterscheidet sich von der Vorrichtung gemäß Fig. 2 im wesentlichen durch die gleichzeitige Verwendung einer Auswaschkolonne B′ und eines "Chlorwasserstoffabspalters" B″ anstelle der alleinigen Verwendung der Kolonne B und durch nachgeschaltete Kolonnen C und D, in welchen das Überkopfprodukt der Hauptkolonne A, bzw. der Seitenstrom der Hauptkolonne A einer destillativen Aufarbeitung unterzogen werden.

Bei der Durchführung des erfindungsgemäßen Verfahrens gemäß der in Fig. 3 dargestellten Ausführungsform gelangen die (201) aus Fig. 2 entsprechenden Reaktionsgase (301) in eine erste Kolonne B′, in welcher sie bei 10° bis 30°C mit einem organischen Lösungsmittel absorbiert werden, wobei durch Erhitzen der Reaktionslösung auf ca. 90°—120°C Chlorwasserstoff und Phosgen über (311) entweichen. Die Phosgen-freie Reaktionslösung, die neben dem Carbamidsäurechlorid Anteile an freiem Isocyanat enthält (302) wird dann mit einem Phenol der erfindungs-gemäß geeigneten Art (303) vereinigt und im Chlorwasserstoffabspalter B″ durch Erhitzen auf ca. 80—150°C unter Abspaltung von Chlorwasserstoff (312) zur Carbamidsäurearylesterlösung (306) umgesetzt, die in der Hauptkolonne, wie bereits oben ausgeführt in eine Phenollösung (307) und eine Monoisocyanatlösung (308) zerlegt wird, wobei die Phenollösung (307) in der Kolonne C in reines Lösungsmittel (309) und aufkonzentrierte Phenollösung (303) (ca. 80 gew.-%ig) und das Kopfprodukt (308) in der Kolonne D in reines Monoisocyanat (305) und reines Lösungsmittel (304) zerlegt werden. Die vereinigten Ströme (304) und (309) bilden den in der Kolonne B′ eingesetzten Lösungsmittelstrom (310).

Die in Fig. 4 dargestellte Vorrichtung unterscheidet sich von der Vorrichtung gemäß Fig. 3 durch die Anordnung der zur Reindarstellung des Verfahrensprodukts dienenden Destillationskolonne D zwischen der Kolonne B′ und dem Chlorwasserstoffabspalter B″, wobei die Kolonne D nicht nur die Funktion der Reindarstellung des in der Hauptkolonne anfallenden Verfahrensprodukts übernimmt, sondern vielmehr auch zur Isolierung des bereits im gasförmigen Phosgenierungsprodukt vorliegenden

Monoisocyanats dient. Bei der Durchführung des erfindungsgemäßen Verfahrens gemäß der in fig. 4 dargestellten Ausführungsform gelangt nun das gasförmige Phosgenierungsgemisch (401), welches ebenso wie die Gemische (201) und (301) der Figuren 2 und 3 im wesentlichen aus Monoisocyanat, Carbamidsäurechlorid, Chlorwasserstoff und überschüssigem Phosgen besteht in die Auswaschkolonne B', in welcher unter Verwendung der Lösungsmittelströme (403) und (409) und unter Abdestillieren des bereits vorliegenden Chlorwasserstoffs und überschüssigem Phosgens (410) bei 90°—120°C) eine Carbamidsäurechlorid und Monoisocyanat enthaltende Lösung (402) gebildet wird. Diese Monoisocyanat und Carbamidsäurechlorid enthaltende Lösung gelangt dann in die Destillationskolonne D, wo sie in Monoisocyanat (411), reines Lösungsmittel (403) und eine konzentrierte Carbamidsäurechloridlösung (404) verlegt wird. Diese Carbamidsäurechlorid-lösung (404) wird mit der konzentrierten Phenollösung (405) vereinigt und im Chlorwasserstoffabspalter B'' bei 80—150°C zu Chlorwasserstoff (412) und Carbamidsäurearylesterlösung (406) umgesetzt. Die Zersetzung der Lösung (406) erfolgt in der Hauptkolonne A wie bereits oben dargelegt, wobei der Seitenstrom (407) benso wie bei der Ausführungsform gemäß Fig. 3 in reines Lösungsmittel (409) und konzentrierte Phenollösung (405) zerlegt wird, und wobei das Überkopfprodukt (408) in den oberen Teil der Kolonne D eingeleitet wird, wobei das in (408) enthaltene Monoisocyanat im Strom (411) in reiner Form erhalten wird, während das in (408) enthaltene Lösungsmittel in (403) bzw. (404) aufgenommen wird.

Gegenüber den bekannten Verfahren des Standes der Technik zur Herstellung von niedrigsiedenden Monoisocyanaten weist das erfindungsgemäße kontinuierliche Verfahren folgende Vorteile auf:

1. Die bei der thermischen Spaltung von Carbamidsäurechloriden in Alkylisocyanate und Chlorwasserstoff störend wirkende Rekombination der Spaltprodukte wird durch Überführung der Carbamidsäurechloride in die erst bei höheren Temperaturen spaltbaren Carbamidsäurearylester völlig ausgeschlossen. Der bei der Bildung der Carbamidsäurearylester entstandene Chlorwasserstoff wird auf einfache Weise vor der Carbamidsäureesterspaltung vollständig entfernt, so daß die Isocyanatausbeuten auch im weiteren Verlauf des Verfahrens nicht durch Rückbildung von Carbamidsäurechlorid erniedrigt werden. (Bei den in Figuren 3 und 4 dargestellten "Chlorwasserstoffabspalter" B'' handelt es sich vorzugsweise um einfache beheizbare Reaktionsgefäße mit aufgesetzten Rückflußkühlern, durch welche der Chlorwasserstoff entweicht).
2. Die Verwendung der genannten erfindungsgemäß eingesetzten Phenole für die Bildung von N-Alkylcarbamidsäurearylestern erlaubt es, beide bei der Rückspaltung der Carbamidsäureester entstandenen Spaltprodukte zusammen mit dem verwendeten Lösungsmittel zu verdampfen. Obwohl beide Spaltprodukte nebeneinander in der Gasphase vorliegen, wird aufgrund der erfindungsgemäßen Trenntechnik die Rekombination der Spaltprodukte nahezu vollständig vermieden.
3. Die Überführung der erfindungsgemäßen Phenole in die Gasphase bedeutet hinsichtlich der Kreisführung des Phenols ein wertvoller Reinigungsschritt. Dadurch wird von vornherein eine Störung des kontinuierlichen Ablaufs des Verfahrens durch hochsiedende oder unter den Reaktionsbedingungen nicht verdampfbare Verunreinigungen ausgeschlossen. Derartige Verunreinigungen bleiben im Sumpf des Carbamidsäureester-Spaltgefäßes zurück und können dort, falls notwendig, auf einfache Weise ausgetragen werden.

Bei den beim erfindungsgemäßen Verfahren einzusetzenden Apparaturen handelt es sich, von den gemachten erfindungswesentlichen Ausführungen abgesehen, um die in der chemischen Verfahrenstechnik üblichen Apparaturen aus den üblicherweise für derartige Umsetzungen geeigneten Materialien.

Die nach dem erfindungsgemäßen Verfahren herstellbaren aliphatischen Monoisocyanate sind wertvolle Ausgangsverbindungen für Pflanzenschutzmittel und Pharmazeutika.

BEISPIEL 1 (Figuren 1 und 2)

2,0 kg (64,5 Mol)/Stunde Methylamin und 9 kg (90,9 Mol)/Stune Phosgen werden jeweils auf ca. 180°C vorerhitzt und bei ca. 350°C in einer Mischkammer zur Reaktion gebracht. Die aus der Mischkammer austretenden Reaktionsgase (201) werden in einer Absorptionskolonne mit 17,5 kg/Stunde einer 40 gew.-%igen Phenollösung in Chlorbenzol, (entsprechend 74,4 Molen Phenol) absorbiert. Die dabei entstandene Reaktionslösung wird durch Erhitzen auf 140°C von Chlorwasserstoff und überschüssigem Phosgen (202) befreit. Die dabei anfallende Lösung (206) bzw. (106) des Carbamidsäurearylesters wird in (101) bei 230°C unter Verdampfen des Lösungsmittels in gasförmiges Monoisocyanat und gasförmiges Phenol gespalten, wobei die Spaltprodukte in die über dem Spaltgefäß (101) angeordnete Destillationskolonne gelangen, die mit einem auf 70°C temperiertem Dephlegmator (104) ausgerüstet ist. Am Kopf der Kolonne werden pro Stunde 3,5 kg (entsprechend 96% der Theorie) reines Methylisocyanat (108) abgezogen, während die unterhalb des Dephlegmators entnommene Phenollösung (107) bzw. (207) im Kreis zur Auswaschkolonne B zurückgeführt wird.

BEISPIEL 2 (Figuren 1 und 3)

124 g (4 Mol)/Stunde Methylamin und 495 g (5 Mol)/Stunde Phosgen werden getrennt auf ca.

200°C vorerhitzt und in einem Reaktionsrohr bei ca. 350°C umgesetzt. Die dabei entstehenden Reaktionsgase (301) werden mit 2000 g Chlorbenzol (310) pro Stunde adsorbiert, wobei man nach Entfernen des Chlorwasserstoffs und des überschüssigen Phosgens (311) eine 11,2 Gew.-% N-Methylcarbamidsäurechlorid und 2,9 Gew.-% Methylisocyanat enthaltende Reaktionslösung (302) erhält. Diese Lösung (302) wird mit 564 g/Stunde einer im Kreis geführten 80 Gew.-%igen Lösung von Phenol in Chlorbenzol (entsprechend 4,8 Molen Phenol) (303) vermischt. Durch Erhitzen auf 80—140°C wird dabei sowohl das Monoisocyanat als auch das Carbamidsäurechlorid, letzteres unter Abspaltung von Chlorwasserstoff (312) in den Carbamidsäurephenylester übergeführt. Die dabei entstehende Carbamidsäureester-Lösung (306) bzw. (106) wird analog Beispiel 1 bei 220°C in dampfförmiges Methylisocyanat, dampfförmiges Phenol und Lösungsmitteldampf zerlegt, wobei pro Stunde oberhalb des 125°C temperierten Dephlegmators (104) am Kolonnenkopf 376 g/Stunde einer 60 Gew.-%igen Lösung von Methylisocyanat in Chlorbenzol (108) bzw. (308) und unterhalb der Kühlvorrichtung als Seitenstrom (107) bzw. (307) 2414 g/Stunde einer 18,5 Gew.-%igen Lösung von Phenol in Chlorbenzol abgenommen werden. Die Methylisocyanatlösung (108) bzw. (308) wird anschließend in einer nachgeschalteten Destillationskolonne in 221 g/Stunde (entsprechend 97% der Theorie) reines Methylisocyanat (305) und Lösungsmittel (304) zerlegt. Die als Seitenstrom (107) bzw. (307) angefallene Phenollösung wird in einer separaten Destillationskolonne durch Abdestillieren von 1850 g/Stunde Chlorbenzol (309) auf einen Phenolgehalt von 80 Gew.-% aufkonzentriert (303) und wiederverwendet. Der Lösungsmittelstrom (309) wird mit dem Lösungsmittelstrom (304) vereinigt und als vereinigter Strom auf (310) wiederverwendet.

BEISPIEL 3 (Figuren 1 und 4)

Beispiel 2 wird dahingehend abgeändert, daß die dort erwähnte N-Methylcarbamidsäurechlorid-Lösung (302) als Lösung (302), vor der Umsetzung mit dem Phenol destillativ vom frei vorliegenden Methylisocyanat (411) befreit wird, wobei im Seitenstrom (404) 523 g/Stunde einer 50 Gew.-%igen Lösung von Carbamidsäurechlorid in Chlorbenzol (entsprechend 2,8 Molen Carbamidsäurechlorid) und im Sumpf (403) 1495 g/Stunde reines Chlorbenzol anfallen. Die konzentrierte Carbamidsäurechlorid-Lösung (404) wurde mit 400 g einer im Kreis geführten 80 Gew.-%igen Lösung von Phenol in Chlorbenzol (entsprechend 3,4 Molen Phenol) (405) vermischt und unter Chlorwasserstoffabspaltung (412) in die zu spaltende Carbamidsäurearylester-Lösung (406 bzw. 106) übergeführt. Bei der Spaltung dieser Lösung (106) bzw. (406) werden pro Stunde 172 g einer 90 Gew.-%igen chlorbenzolischen Methylisocyanat-Lösung (108) bzw. (408) und 664 g einer 50 Gew.-%igen chlorbenzolischen Phenollösung (107) bzw. (407), die noch 1,9 Gew.-% unveränderten Carbamidsäurephenylester enthält, erhalten. Die Methylisocyanatlösung (108) bzw. (408) wird in den oberen Teil der Destillationskolonne D eingeleitet und dort in reines Produkt (411) und Lösungsmittel in (404) bzw. (403) zerlegt. Insgesamt werden 222 g/Stunde entsprechend einer 97%igen Ausbeute an Methylisocyanat gewonnen. Die Phenollösung (107) bzw. (407) wird in einer separaten Destillationskolonne durch abdestillieren von reinem Chlorbenzol (409) auf 80 Gew.-% Phenolgehalt aufkonzentriert und weider in den Phenol-Kreislauf (405) zurückgeführt. Das dabei anfallende Chlorbenzol (409) wird an den Prozeßanfang zurückgeführt.

BEISPIEL 4 (Figuren 1 und 2)

25 kg/Std. einer in Analogie zu Beispiel 1 hergestellten und von HCL und überschüssigem Phosgen befreiten N-Äthylcarbamidsäurephenylester-Lösung in Chlorbenzol, die 48 Gew-% (72,7 Mol) N-Äthylcarbamidsäurephenylester, 3 Gew.-% Phenol und 0,3 Gew.-% Diphenylcarbonat enthält, werden im Spaltgefäß (101) bei 190°C zu gasförmigem Äthylisocyanat und gasförmigem Phenol gespalten. Die Spaltprodukte gelange mit Lösungsmitteldämpfen in die Destillationskolonne, die mit einem Dephlegmator (104) ausgerüstet ist. der mit 15°C — Sole so betrieben wird, daß sich in dem über dem Dephlegmator liegenden Kolonnenboden eine Temperatur von 150°C einstellt. Am Kopf der Kolonne werden 5,56 kg/Std. einer 80 gew.-%igen Äthylisocyanat-Lösung in Chlorbenzol (108) bzw. (208) abgezogen, die in einer weiteren Kolonne (nicht gezeichnet) in Lösungsmittel und 4,45 kg/Std. reines Äthylisocyanat aufgetrennt werden, während unterhalb des Dephlegmators 19,4 kg/Std. einer 34,2 gew.-%igen Phenollösung mit 8,5 Gew.-% N-Athylcarbamidsäurephenylester entnommen (107) bzw. (207) und mit dem obengenannten Lösungsmittelstrom vereinigt und in die Kolonne B zurückgeführt werden. Gleichzeitig fließen über einen oberhalb des Dephlegmators angeordneten Entnahmeboden (nicht gezeichnet) 15 l/Std. eines 2. Seitenstroms in die Spaltvorrichtung zurück.

BEISPIEL 5 (Figuren 1 und 2)

25 kg/Std. einer in Analogie zu Beispiel 1 hergestellten und von HCL und überschüssigem Phosgen befreiten N-Isopropylcarbamidsäurephenylester-Lösung in Chlorbenzol, die 52 Gew.-% (72,6 Mol) N-Isopropylcarbamidsäurephenylester, 2,5 Gew.-% Phenol und 0,4 Gew.-% Diphenylcarbonat enthält, werden im Spaltgefäß (101) bei 160°C jedoch im Unterschied zu den vorhergehenden, bei Normaldurck arbeitenden Beispielen bei einem Vakkum von 250 mbar zu gasförmigen Isopropylisocyanat und gasförmigem Phenol gespalten. Die Spaltprodukte gelangen mit Lösungsmitteldämpfen in die Destillationskolonne, die mit einem Dephlegmator (104) ausgerüstet ist, der so reguliert wird, daß

die Temperatur oberhalb des Dephlegmators 110°C beträgt. Am Kopf der Kolonne werden 6,53 kg/Std. einer 75 gew.-%igen Isopropylisocyanat-Lösung in Chlorbenzol (108) bzw. (208) abgezogen, die in einer nicht gezeichneten weiteren Kolonne in reines Lösungsmittel und 4,9 kg/Std. (entsprechend 92% der Theorie) reines Isopropylisocyanat aufgetrennt werden, während unterhalb des Dephlegmators 18,4 kg/Std. einer 32,8 gew.-%igen Phenollösung mit 14,6 Gew.-% N-Isopropylcarbamidsäurephenylester entnommen (107) bzw. (207) und mit dem reinen Lösungsmittelstrom vereinigt in die Kolonne B zurückgeführt werden. Gleichzeitig schließen über einen oberhalb des Dephlegmators angeordneten Entnahmeboden (nicht gezeichnet) 20 l/Std. eines zweiten Seitenstroms in das Spaltgefäß (101) zurück.

**Patentansprüche**

1. Verfahren zur kontinuierlichen Herstellung von Monoisocyanaten der Formel

R—NCO

in welcher

R für einen gesättigten aliphatischen Kohlenwasserstoffrest mit 1 bis 3 Kohlenstoffatomen steht, durch thermische Spaltung der entsprechenden, in einem unter den Reaktionsbedingungen inerten organischen Lösungsmittel mit einem mindestens 20°C über dem Siedepunkt des zu bildenden Monoisocyanats liegenden Siedepunkt gelösten N-Alkylcarbamidsäurearylester der Formel

R—NH—CO—O—R′

in welcher

R die genannte Bedeutung hat und

R′ für einen Rest steht wie er durch Entfernung der Hydroxylgruppe aus einem Monophenol eines bei Normaldruck unterhalb 250°C liegenden Seidepunkts erhalten wird,

und destillative Auftrennung der Spaltprodukte, dadurch gekennzeichnet, daß

a) man die Lösung des zu spaltenden N-Alkylcarbamidsäurearylesters bei Normaldruck oder bei leicht vermindertem Druck im Bereich von 200 bis 1013 mbar unter Spaltung des Esters in das entsprechende Monoisocyanat und das entsprechende Phenol, sowie unter Verdampfen des Lösungsmittels und der Spaltprodukte auf eine Temperatur im Bereich von 160 bis 250°C erhitzt und

b) man die gemäß a) anfallenden Dämpfe gemeinsam in eine Destillationskolonne leitet, aus welcher man das Phenol und zumindest die Hauptmenge des Lösungsmittels in Form eines gemeinsamen oder in Form mehrerer Seitenströme und das Monoisocyanat gegebenenfalls zusammen mit einem Teil des Lösungsmittels am Kopf gewinnt.

2. Verfahren gemäß Anspruch 1, dadurch gekennzeichnet, daß man eine einen oberhalb eines Seitenstrom-Entnahmebodens angeordneten temperaturregulierbaren Dephlegmator aufweisende Füllkörperkolonne verwendet und die Tempertur des Dephlegmators so wählt, daß die Gesamtmenge des Phenols zusammen mit der Hauptmenge des Lösungsmittels als Seitenstrom unterhalb des Dephlegmators und das Monoisocyanat gegebenenfalls zusammen mit einer Restmenge des Lösungsmittels als Kopfprodukt anfallen.

3. Verfahren gemäß Anspruch 1, dadurch gekennzeichnet, daß man eine einen zwischen zwei Seitenstrom-Entnahmeböden angeordneten, temperaturregulierbaren Dephlegmator aufweisende Füllkörperkolonne verwendet und die Temperatur des Dephlegmators so wählt, daß die Hauptmenge des Phenols als Seitenstrom unterhalb des Dephlegmators und das Monoisocyanat gegebenenfalls mit einer Restmenge des Lösungsmittels als Kopfprodukt anfallen, während Restmengen an, den Dephlegmator passierendem Phenol zusammen mit geringen Anteilen an Isocyanat und Carbamidsäurearylester als Lösung in einer Teilmenge des eingesetzten Lösungsmittels an einer geeigneten Stelle oberhalb des Dephlegmators der Destillationskolonne als zweiter Seitenstrom entnommen und an einer beliebigen Stelle unterhalb des Dephlegmators in die Spaltapparatur zurückgeführt werden.

4. Verfahren gemäß Anspruch 1 und 2, dadurch gekennzeichnet, daß man das als Seitenstrom anfallende Phenol und das als Seitenstrom anfallende Lösungsmittel im Gemisch oder nach destillativer Auftrennung zur Herstellung der als Ausgangsmaterial eingesetzten Lösung durch Auflösen von, bzw. Reaktion mit bei der Gasphasenphosgenierung von Monoaminen der Formel R—$NH_2$ entstehenden Reaktionsgasen wiederverwendet, wobei R die in Anspruch 1 genannte Bedeutung hat.

**Revendications**

1. Procédé de préparation en continu de monoisocyanates répondant à la formule:

R—NCO

dans laquelle

R représente un groupe d'hydrocarbure aliphatique saturé contenant 1 à 3 atomes de carbone, par dissociation thermique de l'ester arylique d'acide N-alkylcarbamique correspondant dissous dans un solvant organique inerte dans les conditions réactionnelles et ayant un point d'ébullition supérieur d'au moins 20°C à celui du monoisocyanate à former, cet ester arylique d'acide N-alkylcarbamique répondant à la formule:

R—NH—CO—O—R'

dans laquelle

R a la signification indiquée ci-dessus, et

R' représente un radical obtenu par élimination du groupe hydroxy d'un monophénol d'un point d'ébullition inférieur à 250°C sous pression normale,

ainsi qu'en séparant les produits de dissociation par distillation, caractérisé en ce que:

a) on chauffe la solution de l'ester arylique d'acide N-alkylcarbamique à dissocier à une température se situant dans l'intervalle allant de 160 à 250°C sous pression normale ou sous une pression légèrement réduite se situant dans l'intervalle allant de 200 à 1.013 mbars avec dissociation de l'ester en monoisocyanate correspondant et en phénol correspondant, ainsi que par évaporation du solvant et des produits de dissociation, et

b) on fait passer ensemble les vapeurs obtenues sub (a) dans une colonne de distillation de laquelle on obtient le phénol et au moins la quantité principale du solvant sous forme d'un courant latéral commun ou de plusieurs courants latéraux, de même que le monoisocyanate éventuellement avec une partie du solvant, en tête.

2. Procédé suivant la revendication 1, caractérisé en ce qu'on utilise une colonne à corps de remplissage comportant un déphlegmateur à température réglable disposé au-dessus d'un plateau de prélèvement d'un courant latéral et on choisit la température de ce déphlegmateur de façon à obtenir la quantité totale du phénol conjointement avec la quantité principale du solvant sous forme d'un courant latéral en dessous de ce déphlegmateur, ainsi que le monoisocyanate éventuellement avec une quantité résiduelle du solvant, comme produit de tête.

3. Procédé suivant la revendication 1, caractérisé en ce qu'on utilise une colonne à corps de remplissage comportant un déphlegmateur à température réglable disposé entre deux plateux deprélèvement de courants latéraux et on choisit la température de ce déphlegmateur de façon à obtenir la quantité principale du phénol sous forme d'un courant latéral en dessous de ce déphlagmateur et le monoisocyanate éventuellement avec une quantité résiduelle du solvant, comme produit de tête, tandis que l'on retire, sous forme d'un deuxième courant latéral, des quantités résiduelles du phénol passant dans le déphlegmateur, conjointement avec de faibles fractions d'isocyanate et d'ester arylique d'acide carbamique sous forme d'une solution dans une quantité partielle du solvant utilisé à un endroit approprié au-dessus du déphlegmateur de la colonne de distillation, pour les recycler ensuite à l'appareil de dissociation à n'importe quel endroit se situant en dessous du déphlegmateur.

4. Procédé suivant l'une quelconque des revendications 1 et 2, caractérisé en ce qu'on réutilise le phénol obtenu sous forme d'un courant latéral, de même que le solvant obtenu également sous forme d'un courant latéral, en mélange ou après séparation par distillation pour la préparation de la solution utilisée comme matière de départ en dissolvant les gaz réactionnels formés lors de la phosgénation de monoamines de formule R—$NH_2$ en phase gazeuse, ou par réaction avec ces gaz réactionnels, R ayant la signification indiquée dans la revendication 1.

**Claims**

1. A process for the continuous production of monoisocyanates of the formula

R—NCO

in which

R represents a saturated aliphatic hydrocarbon radical with 1 to 3 carbon atoms by thermal decomposition of the corresponding N-alkylcarbamic acid aryl esters of the formula

R—NH—CO—O—R'

in which

R has the meaning given and

R' represents a radical of the type obtained by removing the hydroxyl group from a monophenol having a boiling point lying below 250°C at normal pressure,

dissolved in an organic solvent which is inert under the reaction conditions and has a boiling point lying at least 20°C above the boiling point of the monoisocyanate to be formed, and by separation by distillation of the decomposition products, characterised in that

a) the solution of the N-alkylcarbamic acid aryl ester to be split up is heated at normal pressure or at slightly reduced pressure within the range of 200 to 1013 mbar with decomposition of the ester into the corresponding monoisocyanate and the corresponding phenol, as well as with evaporation of the solvent and the decomposition products, to a temperature in the range of 160°C to 250°C and

b) the vapours formed in a) are fed together into a distillation column from which the phenol and at least the main quantity of the solvent are obtained in the form of a combined stream or in the form of several side streams and the monoisocyanate optionally together with a portion of the solvent is removed from the head.

2. A process according to claim 1, characterised in that a packed column having a temperature-controllable dephlegmator arranged above a side stream removal plate is used and the temperature of the dephlegmator is selected in such a way that the total quantity of the phenol together with the main quantity of the solvent are obtained as a side stream below the dephlegmator and the monoisocyanate, optionally together with a residual quantity of the solvent, is produced as head product.

3. A process according to claim 1, characterised in that a packed column having a temperature-controllable dephlegmator arranged between two side stream removal plates is used and the temperature of the dephlegmator is selected in such a way that the main quantity of the phenol is obtained as a side stream below the dephlegmator and the monoisocyanate, optionally with a residual amount of the solvent, is produced as head product, whereas residual quantities of phenol passing through the dephlegmator together with small amounts of isocyanate and carbamic acid aryl ester are removed, in the form of a solution in a part of the solvent used, as a second side stream at a suitable position above the dephlegmator of the distillation column and recycled into the splitting apparatus at any required position below the dephlegmator.

4. A process according to claims 1 and 2, characterised in that the phenol produced as a side stream and the solvent produced as a side stream are reused, either as a mixture or after separation by distillation, for the production of the solution used as starting material by dissolution of or reaction with reaction gases formed during the gaseous phase phosgenation of monoamines of the formula R—$NH_2$, in which R has the meaning given in claim 1.

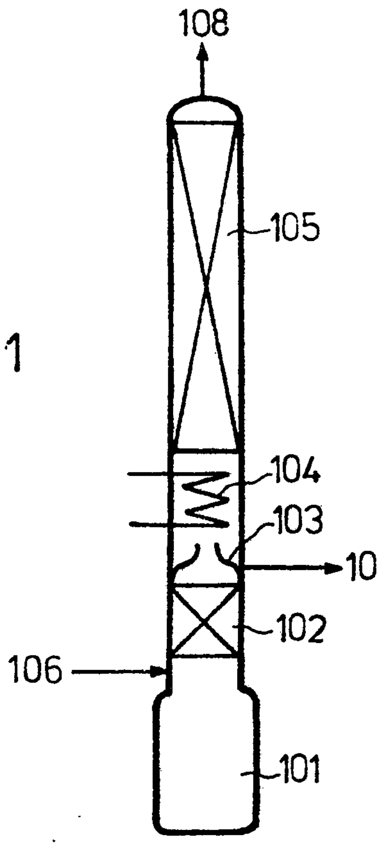


FIG. 1

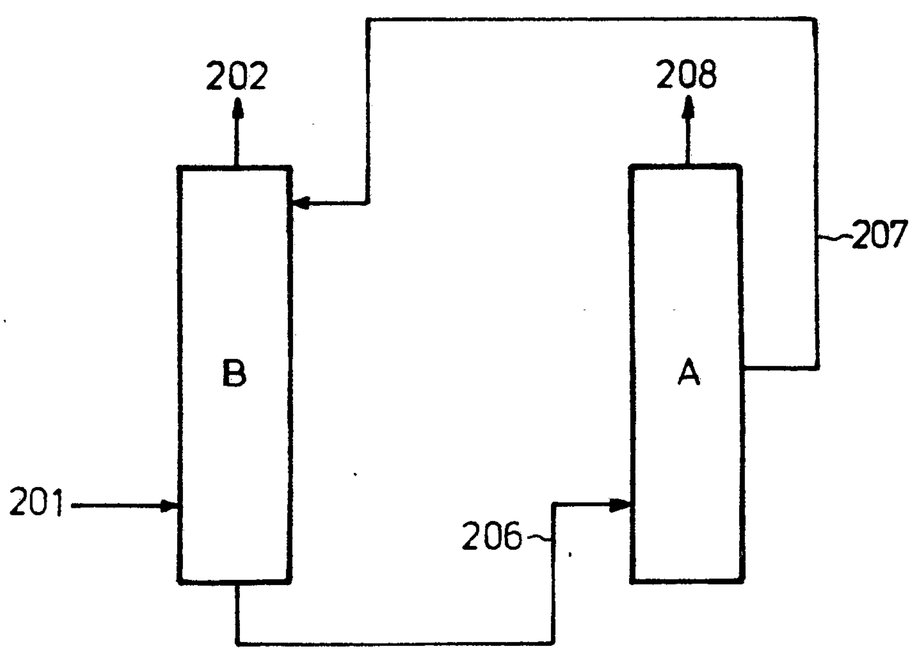


FIG. 2

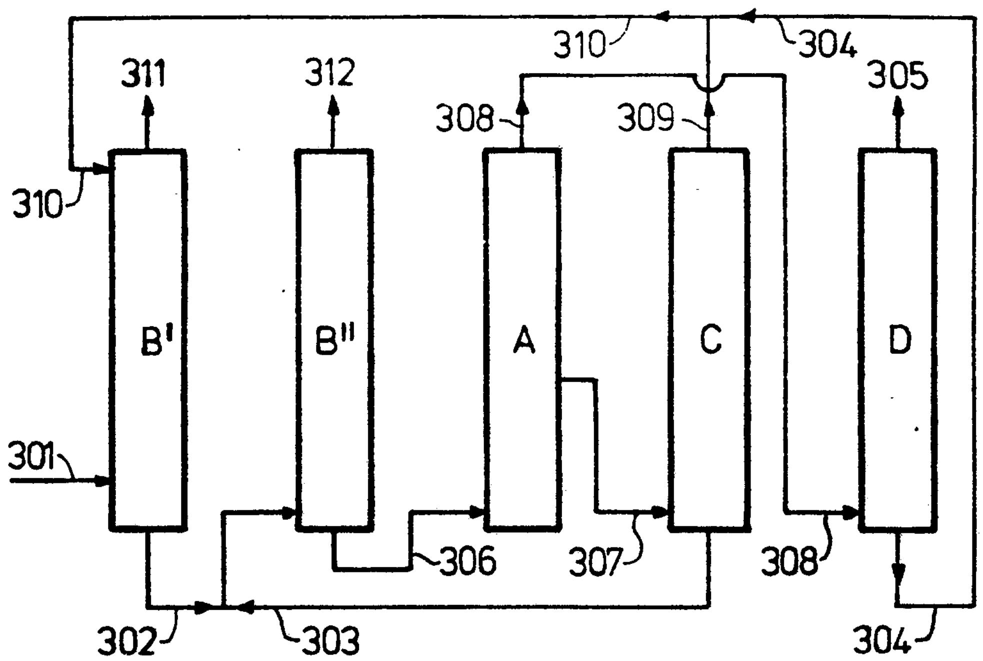
311
312
310
304
305
308
309
310
B'
B''
A
C
D
301
306
307
308
302
303
304

FIG. 3

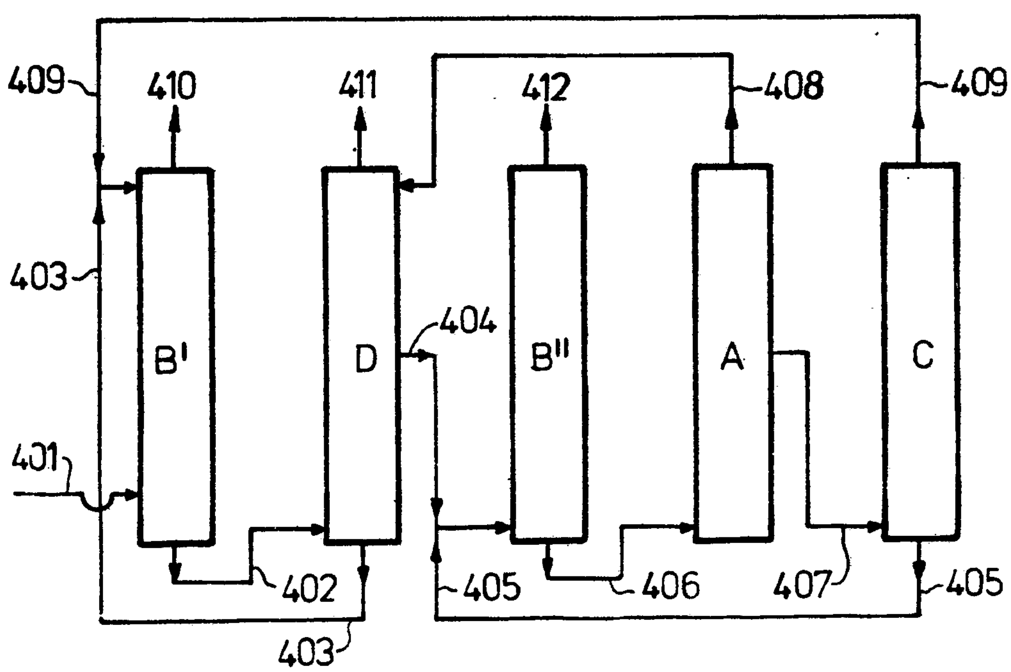
409
410
411
412
408
409
403
404
B'
D
B''
A
C
401
402
405
406
407
405
403

FIG. 4